# EUROPÄISCHE PATENTSCHRIFT

(11) **EP 3 273 234 B1**
(45) Veröffentlichungstag und Bekanntmachung des Hinweises auf die Patenterteilung: **01.12.2021**
(21) Anmeldenummer: 17182611.8
(22) Anmeldetag: 21.07.2017
(51) Int. Cl.: G01N 33/00, G01N 21/64, B01L 3/00, G01N 21/77, G01N 30/88

(54) **PROBENTRÄGER, ANALYSEVERFAHREN SOWIE OPTISCHES MESSGERÄT**
SAMPLE CARRIER, ANALYSIS METHOD AND OPTICAL MEASURING DEVICE
PORTE ÉCHANTILLONS, PROCÉDÉ D'ANALYSE ET DISPOSITIF DE MESURE OPTIQUE

(30) Priorität: 22.07.2016 DE 102016113607
(43) Veröffentlichungstag der Anmeldung: 24.01.2018
(73) Patentinhaber: Bundesrepublik Deutschland, vertreten durch den Bundesminister für Wirtschaft und Energie, 12205 Berlin (DE)
(72) Erfinder: NOSKE, Reinhard, 15827 Blankenfelde (DE); BERNSTEIN, Thomas, 12249 Berlin (DE); SCHLAU, Sven, 15745 Wildau (DE); FILARSKI, Sylvia, 15711 Königs Wusterhausen (DE)
(74) Vertreter: Zimmermann & Partner Patentanwälte mbB

(56) Entgegenhaltungen:
- EP-A1- 2 962 758
- DE-A1- 2 541 031
- US-A1- 2012 230 892
- US-A1- 2012 281 208
- US-A1- 2012 304 729

## Beschreibung

Die seitenrichtige und exakte Positionierung eines Probenträgers in einer Messvorrichtung birgt gerade für solche Probenträger Schwierigkeiten, die entweder eine analytsensitive Schicht aufweisen oder, beispielsweise, einseitig eine flüchtige organische Verbindung zumindest zeitweise tragen oder mit ihr während einer Messung in Wechselwirkung treten. Beispielsweise kann ein Probenträger verdreht, verkantet, seitenverkehrt und somit falsch in eine Messvorrichtung eingelegt werden und gewonnene Messergebnisse können in der Folge verfälscht oder falsch interpretiert werden. US 2012 / 0 304 729 A1 lehrt ein Multianalyt-Detektionssystem und Verfahren zur Bestimmung von Sprengstoffen sowie chemischen und biologischen Verbindungen.

Vor diesem Hintergrund wird eine Vorrichtung nach Anspruch 1, ein Messgerät nach Anspruch 14 und ein Analyseverfahren nach Anspruch 20 vorgeschlagen. Weitere Ausführungsformen, Modifikationen und Verbesserungen ergeben sich anhand der folgenden Beschreibung und der beigefügten Ansprüche.

Gemäß einer ersten Ausführungsform wird ein Probenträger vorgeschlagen, der eine erste Folie, eine zweite Folie und ein starres Substrat umfasst, wobei die erste Folie das starre Substrat entlang einer Umfangsrichtung des starren Substrats umgibt und der Probenträger eingerichtet ist, eine fluidisch dichtende Verbindung in einem Messraum eines Messgerätes auszubilden und den von zwei Modulen des Messgerätes ausbildbaren Messraum fluidisch dichtend in zwei voneinander getrennte Bereiche zu unterteilen.

Vorteile dieser Ausführungsform umfassen die auf kompakter Bauweise beruhende sichere Orientierung und Zuordnung einer Probe vor, während und nach einer Messung. Als besonders vorteilhaft ist die Möglichkeit eines unkomplizierten Wechsels nacheinander zu vermessender Targets bzw. Proben anzusehen, was sich aus dem stets erneut und reproduzierbar erreichbaren fluiddichten Abschluss eines Messraumes durch das selbstjustierende Target ergibt.

Gemäß der ersten Ausführungsform ist die zweite Folie benachbart zum starren Substrat (30) auf der ersten Folie befestigt und überlappt einen Randbereich des starren Substrats (30), wobei die beiden Folien an einander gegenüberliegenden Positionen jeweils eine Öffnung aufweisen.

Vorteile umfassen die Möglichkeit eines zweiseitigen fluiddichten Kontaktes mit Modulen eines geometrisch angepassten Messgeräts.

Gemäß einer weiteren Ausführungsform ist die zweite Folie im Randbereich des starren Substrats (30) mit dem starren Substrat (30) verbunden.

Vorteile dieser Ausführungsform umfassen eine zuverlässige Halterung des targets durch den Träger.

Gemäß einer weiteren Ausführungsform ist ein zentraler Bereich des starren Substrats durch keine der beiden Folien bedeckt.

Vorteile dieser Ausführungsform umfassen die Möglichkeit, das Target in drei unterschiedlichen Messmodi zu analysieren: Auflicht, Durchlicht und Reflektion.

Gemäß einer anderen Ausführungsform ist der zentrale Bereich des starren Substrats auf dessen erster Seite frei von der erstgenannten Folie.

Vorteile dieser Ausführungsform umfassen die Möglichkeit des unmittelbaren Kontaktes einer an die offene Seite herangeführten Verbindung mit einer analytsensitiven Schicht oder einem Depot auf dem starren Substrat.

Gemäß einer weiteren Ausführungsform weist die erstgenannte, das starre Substrat in einer Umfangsrichtung umgebende erste Folie ein Loch auf, das vom starren Substrat verschlossen ist.

Vorteilhaft ergibt sich eine verbesserte Gasdichtheit während der Messung.

Gemäß einer weiteren Ausführungsform ist weist die erste Folie eine Streifenform auf und trägt entlang einer Längsrichtung eine Vielzahl von starren Substraten, sodass der Probenträger einen Gurt fortlaufend aufgereihter starrer Substrate bildet.

Vorteile dieser Ausführungsform umfassen einen problemlosen Wechsel von Targets, die - einem Transportband vergleichbar - aufeinander folgend exponiert und vermessen werden können.

Gemäß einer weiteren Ausführungsform weist die erste Folie einen Transportrand auf.

Vorteile dieser Ausführungsform umfassen die zuverlässige Positionierung eines Targets innerhalb einer Messanordnung. Ergänzt um einen linearen Schrittantrieb, kann das entsprechende Messgerät zur seriellen Bestückung einer Messzelle mit stets frischen Targets versehen werden und so mit dem Messgerät eine Folge von verschiedenen Proben erfasst werden. Besondere Vorteile bietet das für den Vor-Ort-Betrieb unter Feldbedingungen.

Gemäß einer weiteren Ausführungsform umfasst der Transportrand eine Perforation, eine sich periodisch wiederholende Folge einer Eindellung, einer Aufwölbung eines Lochs, einer Ausnehmung und/oder einer Verdickung der erstgenannten Folie.

Vorteile dieser Ausführungsform umfassen die problemlose Linearbewegung des Probenträgers mit einem gezahnten, im Eingriff mit der Folge stehenden Transportrad.

Gemäß einer weiteren Ausführungsform bildet ein gemeinsamer Fügebereich beider Folien eine Auskehlung, die das starre Substrat umlaufend säumt, oder die Auskehlung ist zumindest durch eine der beiden Folien ausbildbar unter Einwirken einer parallel zu einer Flächennormalen des starren Substrats wirkenden Andruckkraft.

Vorteile dieser Ausführungsform umfassen die Ausbildung einer umlaufenden Dichtfläche und der so ausbildbare zumindest einseitig dichtende fluidisch dichte Kontakt.

Gemäß einer weiteren Ausführungsform ist mindestens eine der beiden Folien chemisch inert; umfasst zumindest eine der beiden Folien ein thermoplastisches Polymer, das ausgewählt ist unter einem Polyethylen, einem Polypropylen, und/oder einem Polytetrafluoroethylen und/oder einem Polyamid; und ist zumindest die das starre Substrat umgebende erste Folie lichtundurchlässig. Diese Lichtundurchlässigkeit der Folie ist beispielsweise durch Zusatz eines Pigments, bevorzugt durch Zusatz von Ruß erreichbar.

Vorteile dieser Ausführungsform umfassen das Unterbinden der Einwirkung von Fremdlicht und die Verhinderung der Verschleppung von Fremdstoffen durch die Folie in den Messraum. Die Zuverlässigkeit der reproduzierbar erhebbaren Messwerte ist gesteigert. Ein Signal-/Rausch-Verhältnis ist maximiert.

Gemäß einer weiteren Ausführungsform umfasst eine vom Probenträger getragene Probe eine für einen Analyten sensitive Schicht, alternativ Sensorschicht genannt.

Vorteile dieser Ausführungsform umfassen die kontaminationsfreie Handhabbarkeit der üblicherweise berührungsempfindlichen analytsensitiven Schichten.

Gemäß einer anderen Ausführungsform ist die Sensorschicht in Teilbereiche untergliedert, die eine unterschiedliche Sensitivität gegenüber unterschiedlichen Analyten aufweisen.

Vorteilhaft können so gleichzeitig mehre Analyten aus einem Gasstrom erfasst bzw. nachgewiesen werden. Beispielsweise wird die Sensorschicht so angepasst, dass unterschiedliche Quadranten der Sensorschicht unterschiedliche Sensitivitäten für ein und denselben Analyten aufweisen. Hieraus ergibt sich die vorteilhafte Möglichkeit der Quantifizierung ein und desselben Analyten. Werden in unterschiedlichen Quadranten unterschiedliche Fluoreszenzfarbstoffe eingesetzt, so können vorteilhaft mehrere Analyte aus einem Gasstrom parallel nachgewiesen werden.

Gemäß einer weiteren Ausführungsform ändert sich eine Fluoreszenzeigenschaft der für den Analyten sensitiven Schicht in Abhängigkeit von einer Art und/oder einer Konzentration des Analyten reproduzierbar. Das starre Substrat kann vorteilhaft ausgewählt sein zwischen einem Natriumsilikat- und einem Borosilikat-Glas. Es trägt die für den Analyten sensitive Schicht.

Vorteilhaft sind derartige starre Substrate für Fluoreszenz-Messungen geeignet, absorbieren selbst in den relevanten Wellenlängenbereichen nicht und erlauben somit die hochgenaue Messung auch kleinster Konzentrationen der jeweiligen Analyte.

Gemäß einer weiteren Ausführungsform umfasst die vom Probenträger getragene Probe ein Depot für eine flüchtige organische Verbindung (VOC). Hierbei wird das Depot von einer auf dem starren Substrat angeordneten porösen Schicht gebildet.

Vorteile dieser Ausführungsform umfassen die, ggf. auch bei gezielter Temperaturführung ermöglichte lokale Sublimation und Anreicherung einer Probe aus einem Gasstrom.

Gemäß einer weiteren Ausführungsform umfasst der Probenträger eine dem starren Substrat zugeordnete Bezeichnung. Die Bezeichnung kann alphanumerisch sein aber auch digital auslesbar, beispielsweise ein Strichcode sein.

Vorteile dieser Ausführungsform ergeben sich daraus, dass verschiedene starre Substrate mit ihren jeweiligen analytsensitiven Schichten oder Depots auf einem Gurt voneinander unterscheidbar sind, auch wenn sie visuell einander stark ähneln oder überhaupt nicht unterscheidbar wären.

Gemäß einer weiteren Ausführungsform wird ein Messgerät vorgeschlagen, das zwei beweglich zueinander angeordnete Messgerätmodule umfasst, die im Kontakt mit einem der vorstehend beschriebenen Probenträger einen Kanal zum Führen eines Fluids und eine von den zwei Messgerätemodulen gebildete Messkammer ausbilden, wobei der Kanal eine Ausnehmung einer Kanalwand aufweist, und die Messkammer durch den Probenträger fluidisch dichtend in zwei voneinander getrennte Bereiche geteilt wird.

Vorteile dieser Ausführungsform umfassen eine erleichterte Zugänglichkeit des Kanals. Eine Kontrolle auf eventuelle Verunreinigungen und deren Entfernung ist problemlos möglich. Andererseits erlaubt die ausgebildete fluidisch dichtende Verbindung eine von Störsignalen weitestgehend freie Messung.

Gemäß der vorgenannten Ausführungsform umfasst jeder Teil der fluidisch dichtend geteilten Messkammer zumindest eine optische Komponente, wobei letztere ausgewählt ist unter einem Spiegel, einer Lichtquelle, und/oder einem Lichtsensor.

Vorteile umfassen die Möglichkeit eine eventuell ausgefallene Komponente problemlos austauschen zu können, oder durch einfaches Wechseln, beispielsweise einer als Lichtquelle verwendeten LED, den Wellenlängenbereich eines Anregungslichtes problemlos der jeweiligen Messaufgabe anpassen zu können.

Gemäß der vorstehend beschriebenen Ausführungsform weist die in einem der beiden Messgerätmodule angeordnete Komponente jeweils in Richtung des anderen Messgerätmoduls der vom Target im Probenhalter fluidisch dichtend geteilten Messkammer.

Vorteile umfassen die Möglichkeit der Durchführung von optischen Messungen im Durchlichtmodus.

Gemäß einer weiteren Ausführungsform weist einer der beiden Teile der fluidisch dichtend geteilten Messkammer ein Messfenster auf.

Vorteile dieser Ausführungsform umfassen den Schutz der jeweiligen, hinter dem Messfenster angeordneten optischen Komponente(n) vor Staub, Feuchtigkeit- und Temperaturschwankungen und/oder mechanischer Berührung. Das kann besonders für einen Spiegel von Bedeutung sein.

Gemäß einer weiteren Ausführungsform umfasst einer der beiden Messgerätmodule eine Transportvorrichtung für den zuvor beschriebenen Probenträger.

Vorteile dieser Ausführungsform umfassen den geregelten Transport eines Targets in den Messraum und wieder heraus, sowie den raschen und problemlosen Wechsel einer Folge von Targets.

Gemäß einer weiteren Ausführungsform ermöglicht das Zusammenwirken der beiden Messgerätmodule mit dem Probenträger einen gasdichten Kontakt zwischen einem im Kanal strömenden Fluid und einer der Ausnehmung zugewandten Seite des Probenträgers.

Vorteile dieser Ausführungsform umfassen die Vermeidung von Umwelteinflüssen auf die Messergebnisse.

Gemäß einer weiteren Ausführungsform ist die Transportvorrichtung so angepasst, beispielsweise mit einem Schrittmotor und/oder einer Motorsteuerung versehen, sodass ein diskontinuierlich linearer Vorschub eines starren Substrats in das oder aus dem Messgerät hinein bzw. hinaus ermöglicht wird.

Vorteile dieser Ausführungsform umfassen den raschen und störungsfreien Wechsel mehrerer aufeinander folgender Targets und damit die Messung unter FeldBedingungen an unterschiedlichsten Probenahmeorten.

Gemäß einer weiteren Ausführungsform, umfasst das Zusammenwirken der beiden Messgerätemodule und des Probenträgers drei Zustände. Insbesondere ist in einem ersten Zustand der Probenträger frei beweglich zwischen den beiden Messgerätemodulen angeordnet. In einem zweiten Zustand ist der Probenträger zur Ausnehmung und/oder zum Messfenster justiert angeordnet. Schließlich ist in einem dritten Zustand ein gasdichter Kontakt zwischen der Kanalwand und einer der Ausnehmung zugewandten Seite des Probenträgers herstellbar.

Vorteile dieser Ausführungsform umfassen den Kontakt der analytsensitiven Schicht ausschließlich mit Analyten, die im Kanal geführt werden. Fremdgase, beispielsweise aus der Umgebung, kommen nicht in Kontakt mit der analytsensitiven Schicht, sodass beispielsweise eine von Umwelteinflüssen unbeeinflusste Analyse einer (mit einem temperierten Trägergasstrom) im Kanal geführten (flüchtigen) organischen Verbindung (VOC) möglich ist.

Gemäß einer weiteren Ausführungsform wird eine Verwendung des zuvor beschriebenen Probenträgers vorgeschlagen, die ein gezieltes Anströmen einer Vorderseite des starren Substrats mit einem in einem Kanal eines Messgerätes geführten Trägergasstromes ermöglicht, wobei dem gezielten Anströmen ein fluidisch dichtendes Anpressen einer das starre Substrat umlaufenden Auskehlung an eine ebene Fläche einer Kanalausnehmung vorausgeht und die Auskehlung von der erstgenannten Folie und/oder einer zweiten Folie ausgebildet ist/wird. Hierbei wird unter einer Ausnehmung der Kanalwand ein Loch bzw. eine Ausnehmung in einer Wand des durch zumindest einen oder die beiden Module gebildeten Kanals verstanden.

Vorteile dieser Ausführungsform umfassen die von Störeinflüssen weitestgehend unbeeinflusste Messung verschiedener Proben unter sonst gleichen Bedingungen, d.h. ohne Störeinflüsse von außen.

Gemäß einer anderen Ausführungsform umfasst die Verwendung zusätzlich ein selbständiges Justieren des starren Substrats in und/oder zu der Kanalausnehmung beim Anpressen.

Vorteile dieser Ausführungsform ergeben sich mit der Möglichkeit auf einem Target - beispielsweise in unterschiedlichen Quadranten ein und desselben Targets - mehrere Parameter eines Analyten oder mehrere Analyten parallel erfassen zu können. Beispielsweise kann die Genauigkeit einer semiquantitativen Analyse gesteigert werden, wenn verschieden sensitive Abschnitte der Targetoberfläche parallel ausgelesen werden.

Gemäß einer anderen Ausführungsform umfasst die Verwendung zusätzlich ein Lösen des starren Substrats von der Kanalausnehmung, und ein Ersetzen des starren Substrats durch ein anderes starres Substrat.

Vorteile dieser Ausführungsform umfassen die Möglichkeit einer seriellen Messung. Insbesondere bietet das Vorteile für die Methodenentwicklung im Forschungsbereich.

Gemäß einer anderen Ausführungsform umfasst das Ersetzen hierbei zumindest abschnittsweise ein lineares Transportieren des Probenträgers mit Hilfe eines an der Folie ausgebildeten Transportrandes.

Vorteile dieser Ausführungsform wurden bereits kurz erwähnt und werden in der Beschreibung nachfolgend noch erläutert.

Gemäß einer anderen Ausführungsform umfasst der Transportrand eine Perforation und/oder eine sich periodisch wiederholende Folge einer Eindellung und/oder einer Aufwölbung und/oder einer Ausnehmung und/oder einer Verdickung. Das wurde im Zusammenhang mit dem Probenträger bereits erläutert.

Vorteile dieser Ausführungsform umfassen die Robustheit der bezeichneten Art von Transporträndern. Besonders bewährt sind Perforationen aus dem artfremden Bereich der Kinematografie.

Gemäß einer anderen Ausführungsform steht ein Abschnitt des Transportrandes mit einem Antriebsrad einer diskontinuierlich aktiven Antriebsvorrichtung im Eingriff, sodass ein diskontinuierlich linearer Vorschub der Folie mit dem darauf angeordneten starren Substrat erfolgt.

Vorteile dieser Ausführungsform umfassen die Möglichkeit zum automatischen Betrieb der Messvorrichtung.

Gemäß einer weiteren Ausführungsform wird ein Analyseverfahren vorgeschlagen, umfassend:
- Bereitstellen eines in einem Kanal geführten Fluidstromes,
- fluidisch dichtendes Kontaktieren eines starren Substrats umfassend eine Probe mit dem Fluidstrom, wobei das starre Substrat auf einem Probenträger gemäß den Ansprüchen 1 bis 13 befestigt ist, und eine erste Folie des Probenträgers eine lösbare und fluidisch dichtende Verbindung an einer Kanalausnehmung des Kanals herstellt; und
- qualitatives und/oder quantitatives Bestimmen eines im Fluidstrom vorliegenden Analyten.

Vorteile dieser Ausführungsform umfassen die breite Einsetzbarkeit des Verfahrens für unterschiedlichste Analyte und Messsituationen.

Gemäß einer weiteren Ausführungsform ist beim vorgeschlagenen Analyseverfahren das Fluid des Fluidstromes ein Gas und der Fluidstrom somit ein Trägergasstrom.

Vorteile dieser Ausführungsform umfassen die Verwendbarkeit des Verfahrens bei der Analyse von flüchtigen organischen Verbindungen (VOC), wie z.B. bei der Analyse von bzw. der Suche nach Sprengstoffen.

Gemäß einer weiteren Ausführungsform umfasst das qualitative und/oder quantitative Bestimmen gemäß dem Analyseverfahren: ein Exponieren des starren Substrats mit einer Anregungsstrahlung einer Lichtquelle und ein Erfassen einer vom starren Substrat abgegebenen Emissionsstrahlung mit einem Lichtsensor. Dabei liegt der Analyt bereits im anströmenden Trägergasstrom vor, die vom starren Substrat getragene Probe ist ein analytsensitiver Fluoreszenzfarbstoff und das starre Substrat umfasst somit eine Sensorschicht und ist ein Target.

Alternativ umfasst das qualitative und/oder quantitative Bestimmen gemäß dem Analyseverfahren: das Exponieren des den Kanal verlassenden Trägergasstromes gegenüber einem analytsensitiven Sensor, der kalibriert, getestet oder trainiert werden soll.

Hierbei liegt ein bekannter Analyt, ggf. auch mit einer bekannten Flächenkonzentration, auf dem starren Substrat gebunden (z.B. adsorbiert) vor, wird von dem im Kanal strömenden (chemisch inerten) Trägergasstrom (z.B. künstliche Luft) erfasst und zu einem Ausgang des Kanals getragen, an dem der analytsensitive Sensor (z.B. auch eine Hundenase) platziert ist.

Vorteile dieser Ausführungsform umfassen die Möglichkeit der Präsentation verschiedener Substanzen für das Training eines Spürhundes oder zur Kalibrierung eines anderen Sensors.

Gemäß einer weiteren Ausführungsform umfasst das Analyseverfahren weiter ein Temperieren des Trägergasstromes und/oder eines Messgerätmoduls, der im Kontakt mit dem Probenträger steht.

Vorteile dieser Ausführungsform umfassen das gezielte Unterbinden einer Sublimation oder Adsorption des Analyten an der Kanalwand. Sämtlicher, am Kanaleingang in den Trägergasstrom gelangender Analyt wird zur analytsensitiven Schicht transportiert und wird dort einer Analyse unterzogen. Das Vermeiden von Verlusten während des Transports im Kanal ermöglicht zusätzlich zur qualitativen Analyse eine reproduzierbar quantitative Analyse.

Gemäß einer weiteren Ausführungsform beträgt eine Temperatur des Trägergasstromes und/oder des starren Substrats von 20 bis 75 °C.

Vorteile dieser Ausführungsform ergeben sich aus der Natur der zu erfassenden Analyten. Die Temperatur des Trägergasstromes wird typischerweise dem Dampfdruck bzw. der Flüchtigkeit der Analyten angepasst. Dabei werden flüchtige organische Substanzen detektiert. Diese müssen zum Zeitpunkt der Messung im gasförmigen Zustand vorliegen, sodass die Temperatur des Trägergasstroms ein Verflüchtigen der Substanzen vom Träger bewirkt. Die analysierten Substanzen sind vor allem Very Volatile Organic Compounds (VVOC), Volatile Organic Compounds (VOC) und Semi Volatile Organic Compounds (SVOC).

Gemäß einer weiteren Ausführungsform liegt eine optische Anregungsstrahlung, mit der der Probenträger belichtet wird in einem Wellenlängenbereich von 295 nm bis 800nm.

Vorteile dieser Ausführungsform umfassen die Möglichkeit eine breite Palette von Fluoreszenzindikatoren verwenden zu können. Dem Fachmann sind die innerhalb der jeweiligen Wellenlängenbereiche absorbierenden Substanzklassen vertraut.

Gemäß einer weiteren Ausführungsform liegt eine von der Sensorschicht emittierte Strahlung in einem Wellenlängenbereich von 350 nm bis 850 nm.

Vorteile dieser Ausführungsform umfassen die Möglichkeit eine breite Palette von Fluoreszenzindikatoren verwenden zu können. Dem Fachmann sind die innerhalb der jeweiligen Wellenlängenbereiche emittierenden Substanzklassen vertraut.

Die beschriebenen Ausführungsformen können beliebig miteinander kombiniert werden.

Vorteilhaft können die vorstehend bezeichneten Ausführungsformen der Präsentation einer Probe gegenüber einem Trägergasstrom sowohl in einer Labor-Umgebung als auch im Feldeinsatz genutzt werden. Gerade letzteres ist für die Belange der mobilen vor-Ort-Analyse ein großer Vorteil. Beispielsweise können im Straßenverkehr, bei Massenveranstaltungen oder nach Schadensfällen operativ erforderliche Analysen unter standardisierten Bedingungen vorgenommen werden.

Weiterhin ist es mit der vorgeschlagenen, alternativ auch als "Transport- und Messfassung für Fluoreszenz-Targets" bezeichneten Ausführungsvariante eines Probenträgers möglich, den Nutzungsverlauf von der Produktion des Probenträgers über seine Lagerhaltung bis zum Einsatz und der Entsorgung zu dokumentieren. Die räumlich getrennte, geordnete und durch ggf. maschinell auslesbare Kodierung (Bezeichnung) wiederauffindbar gekennzeichnete Konfektionierung der Probenträger bzw. Targets in den Probenträger verhindert eine Verwechslung der Targets - sowohl während der unmittelbaren Verwendung, - als auch in der Phase der Dokumentation. Sie ist im Forschungsbereich, beim Militär sowie bei allen Anwendern, die einhändig bedienbare (hand-held) Detektoren zum Aufspüren von flüchtigen organischen Verbindungen, beispielsweise von Explosivstoffen oder anderen Umweltgiften in der Luft nutzen, einsetzbar. Damit können die hoch empfindlichen Targets auch bei Messungen in öffentlichen Gebäuden, bei Personenkontrollen, in Verkehrsmitteln, beim Munitionsbergungsdienst sowie bei der Spürhundeausbildung schnell ausgetauscht werden.

Bisher sind die Fluoreszenz-Targets nur schwer manuell handhabbar. Das Einführen in die Messapparatur ist zeitaufwendig und bisher nicht oder nur eingeschränkt automatisierbar. Fehler können insbesondere durch Verwechslungen und Vertauschen von Vorder- und Rückseite der Targets entstehen. Es ist bisher nicht sinnvoll, verschiedene Fluoreszenzschichten, die zum Nachweis unterschiedlicher Chemikalien dienen, auf einem Target unterzubringen, da eine unzureichend Justage des Targets (des Probenträgers mit der Probe) zum optischen Sensor zu Fehlinterpretationen führt.

Die Fassung der Targets erleichtert deren Handhabbarkeit. Einzelne Targets können durch das Aufbringen einer Bezeichnung auf die Transport- und Messfassung unterschieden werden. Die Fluoreszenz-Schichtseite kann ohne optische Hilfsmittel zugeordnet werden. Mehrere Targets können in den Messfassungen zu einem Gurt aufgereiht werden. Dieser Fertigungsvorgang ist automatisierbar. Der Gurt kann mit einem Transportstreifen (Transportrand) versehen werden, mit dem der Probenträger durch das Messsystem bewegt wird. Damit ist ein automatisierter Target-Wechsel möglich und ein Messsystem für den Dauerbetrieb einer Messanordnung unter stets gleichen (Standard-) Bedingungen realisierbar.

In bekannten Messaufbauten muss der Messkanal gegenüber Fremdlicht und Lecks mittels aufwendig gestalteten Dichtlippen geschützt werden. Da die Fassung beide Funktionen übernimmt, können entsprechende Module eines jeweiligen Messgeräts, beispielsweise ein Messkopf, konstruktiv stark vereinfacht und robust ausgeführt werden.

Die Geometrie an der Fassungskante zentriert das Target, sodass dieses korrekt gegenüber dem optischen System positioniert wird. Damit wird die Auswertung unterschiedlicher Fluoreszenzschichten auf einem einzigen Target mittels Mehrfach-Sensor möglich.

Vorteilhaft ist die vorgeschlagene Vorrichtung kostengünstig aus kommerziell problemlos zugänglichen Materialien herstellbar.

In der Vergangenheit wurden komplizierte Messköpfe aufgebaut, bei denen die Handhabung schwierig war. Ein seitenverkehrter Einbau der Targets führte zu Fehlmessungen. Gealterte Targets müssen regelmäßig erneuert werden.

Die Aufgabe der vorliegenden Erfindung ist eine benutzerfreundliche und zuverlässige Gestaltung des Probenträgers, der Messanordnung und ihrer Anwendung. Weiter sollen Herstellung, Lagerung und Vertrieb von Verschleißmaterial vereinfacht werden und die Gefahr einer Fehlbedienung verringert werden.

Die beiliegenden Zeichnungen veranschaulichen Ausführungsformen und dienen zusammen mit der Beschreibung der Erläuterung der Prinzipien der Erfindung. Die Elemente der Zeichnungen sind relativ zueinander und nicht notwendigerweise maßstabsgetreu. Gleiche Bezugszeichen bezeichnen entsprechend ähnliche Teile.

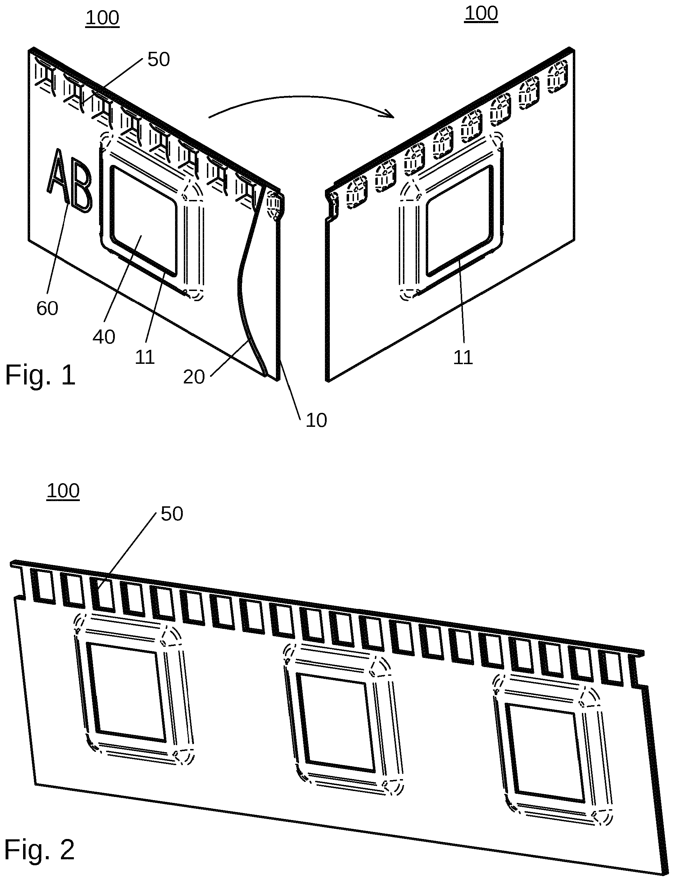

Insbesondere zeigt **Fig. 1** einen thermisch aus zwei Polymerstreifen 10 und 20 geformten Probenträger 100 in Form eines Streifens. Links ist ein Ausschnitt eines von einer ersten Folie 10 und einer zweiten Folie 20 geformten Gurts mit einem Transportrand 50 zu sehen, der thermisch eingeprägt wurde. Die Bezeichnung 60 des Targets 40 ist einseitig sichtbar oder maschinell auslesbar aufgebracht. Sie kennzeichnet damit einerseits die Schichtseite - d.h. diejenige Seite des starren Substrats 30, welche unmittelbar mit der analytsensitiven Schicht 41 versehen ist oder umfasst andere, das Target 40 betreffende Informationen. Derartige Informationen können eine Chargennummer, eine Flächenkonzentration, eine Probennummer oder anderes umfassen.

Wie in **Fig. 2** dargestellt, kann die mit Hilfe des Transportrandes ermöglichte Funktion einer gezielten linearen Bewegung des Probenträgers 100 auch durch einen mittels Perforation erzeugten Transportrand 50 realisiert werden. Fig. 2 zeigt insbesondere einen als Gurt ausgeführten Probenträger 100.

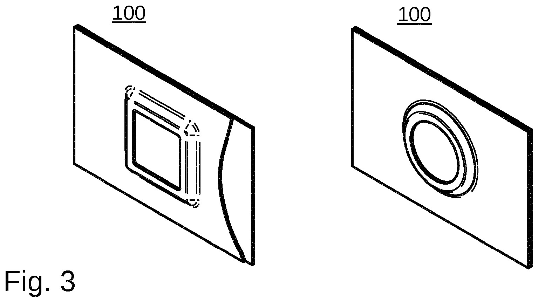

Wie in **Fig. 3** gezeigt, muss der Probenträger 100 nicht notwendigerweise über einen Transportrand 50 verfügen oder die Form des Targets zwingend rechteckig sein.

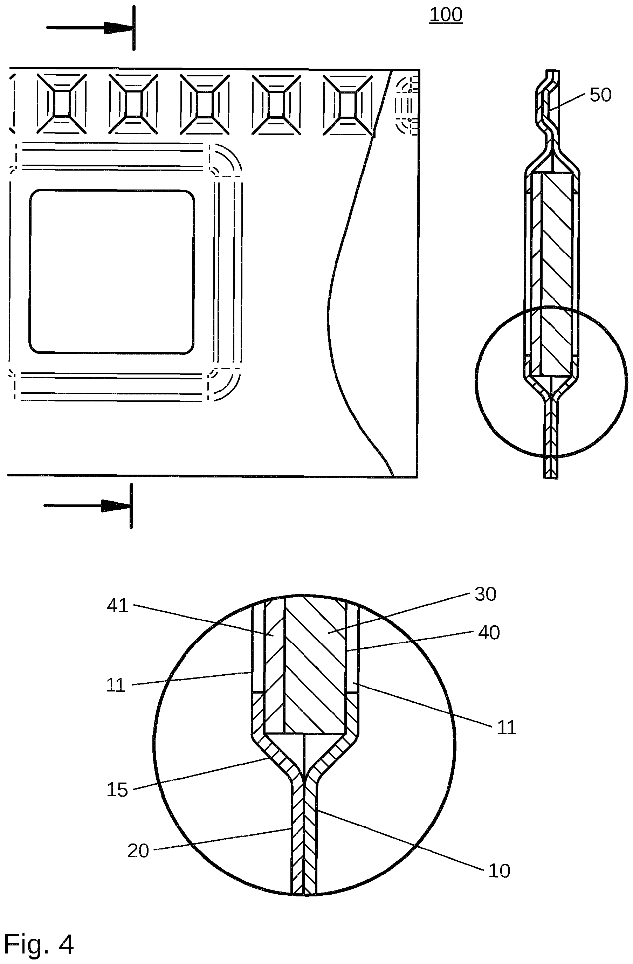

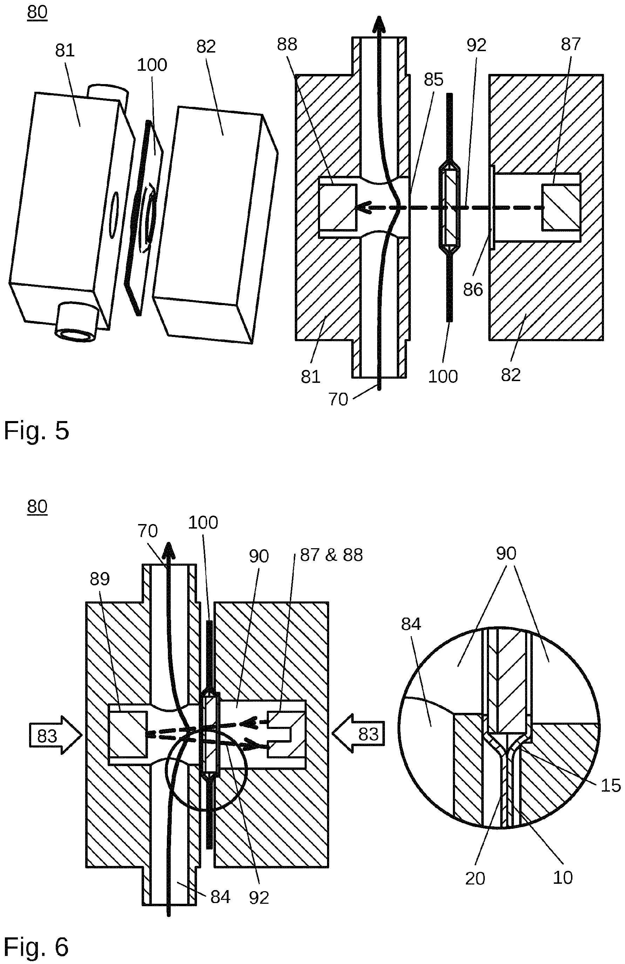

**Fig. 4** stellt den Probenträger 100 bzw. die Transport- und Messfassung mit Target 40 im Schnitt dar. Der Probenträger umfasst gemäß der gezeigten Ausführungsform zwei miteinander Form und stoffschlüssig verbundene Elastomer-Streifen 10 und 20, zwischen die das Target 40 eingebettet ist. Der Transportrand 50 ist geprägt. Wenigstens die dem (Träger-)Gasstrom 70 zugewandte Seite des streifenförmigen Probenträgers 100, wie beispielhaft in der Messanordnung in **Fig. 5** angeordnet, umfasst ein elastisches, chemisch inaktives, licht- und gasundurchlässiges Polymer.

Ein solches Polymer ist typischerweise ein Thermoplast oder Plastomer, beispielsweise ein Polyethylen (PE), ein Polypropylen (PP), ein Hochdruck-PE - synonym auch als Weich-PE bezeichnet und hier abgekürzt als LDPE, oder ein Niederdruck-PE - synonym auch als Hart-PE bezeichnet und hier abgekürzt als HDPE. Ein ebenfalls geeignetes Material für zumindest einen der Streifen des Probenträgers 100 ist ein Polytetrafluoroethylen (PTFE). Kommerziell verfügbare Folien, umfassend PE, PP, LD-PE und HD-PE sind typischerweise kostengünstiger gegenüber PCTE. Die bezeichneten Kunststoffe sind chemisch inert und gehen keine Wechselwirkung mit der Probe bzw. dem Analyten ein.

Bevorzugt umfasst beispielsweise zumindest die dem (Träger-)Gasstrom 70 zugewandte Folie, wie beispielsweise der in der Messanordnung in Fig. 5 gezeigte Probenträger 100, ein elastisches, chemisch inaktives, licht- und gasundurchlässiges Polymer. Bevorzugt ist sowohl jede Folie für sich allein genommen, als auch jegliche zu deren Verbindung verwendete Fügemittel, etwa Klebstoffe, lichtundurchlässig. Für die Verwendung der beschriebenen Transport- und Meßfassung, die hier synonym als Probenträger 100 bezeichnet wird, muss im Zusammenhang mit fluoreszenzoptischen Messungen die Folie, die nicht mit dem zu analysierenden Medium in Berührung kommt, lediglich lichtundurchlässig oder lichtdicht sein. In diesem Zusammenhang wird unter lichtdicht oder lichtundurchlässig ein solches Material verstanden, das zumindest undurchlässig ist für Licht mit einer Wellenlänge, die innerhalb des für die fluoreszenzoptische Messung verwendeten Wellenlängenbereichs liegt. Bevorzugt ist das Folienmaterial undurchlässig im Bereich UV-vis bis nahes IR. Typischerweise für fluoreszenzoptische Messungen verwendete Wellenlängen für die Fluoreszenzanregung bzw. Fluoreszenzmessung (Emission) liegen im Bereich von 295 nm bis 800 nm, beispielsweise zwischen 350 nm und 850 nm.

Die erste Folie 10 und die zweite Folie 20 sind typischerweise durch Verschweißen oder Verkleben so miteinander verbunden, dass in Umfangsrichtung des Targets 40 mindestens einseitig eine Auskehlung 15 bzw. Schräge (engl. - chamfer) entsteht, die einerseits der exakten Ausrichtung zum oder im Messraum eines Messgerätes dienen kann. Andererseits kann die Auskehlung 15 die Funktion einer Dichtlippe erfüllen. Wenn der Probenträger 100 beispielsweise im Durchlichtverfahren eingesetzt werden soll, so weisen beiden Folien an einander gegenüberliegenden Positionen jeweils eine Öffnung 11 auf. Diese Öffnungen können beispielsweise aus der jeweiligen Folie ausgeschnitten oder ausgestanzt werden. Falls das Target im Auflichtverfahren nicht durchstrahlt werden soll, kann in einer Folie auf die zweite Öffnung verzichtet werden.

Wie in **Fig. 6** dargestellt, liegt der Probenträger 100 an den vom Messstrahl durchleuchteten Seiten dicht an der Ausnehmung im Kanal an und verhindert das Eindringen von Fremdlicht bzw. den ungewollten Gasaustausch mit der Umgebung. Die Dichtigkeit wird durch eine Anpresskraft 83 erzeugt. Damit weist im Fall einer Klebeverbindung die dem Gasstrom 60 zugewandte analytsensitive Seite 41 des Probenträgers 100 vorteilhaft keine Klebeschicht zum Target 40 auf. Wenn der Probenträger 100 im Durchlichtverfahren verwendet werden soll, so sind - werden zwei Folien 10 und 20 verwendet - in beiden Folien einander gegenüberliegende Öffnungen 11 ausgeschnitten. Falls das Target im Auflichtverfahren nicht durchstrahlt werden soll, kann auf eine der beiden Öffnungen - nämlich auf die auf der von der Sonde abgewandten Seite, verzichtet werden.

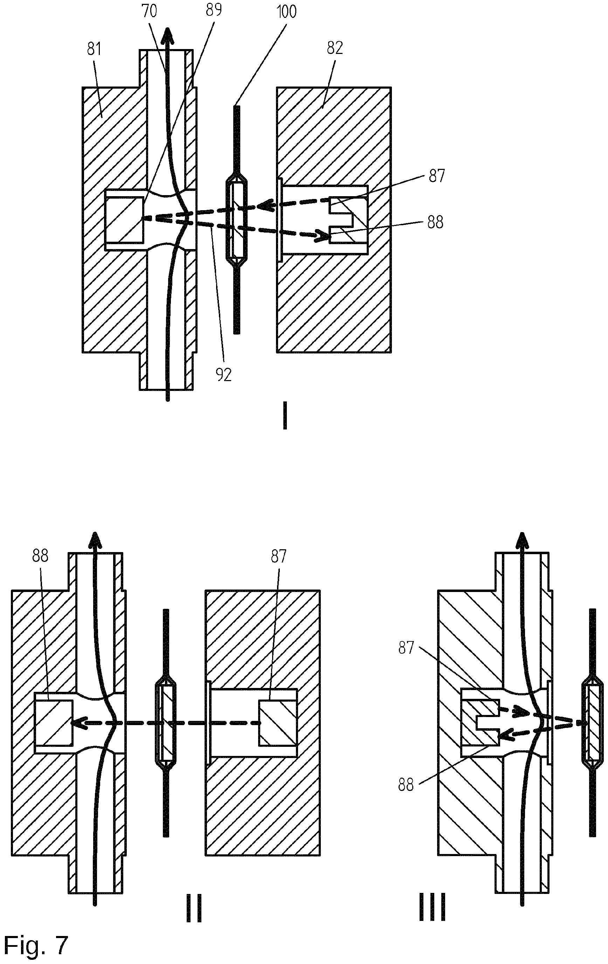

**Fig. 7** zeigt Explosions-Zeichnungen von unterschiedlichen Messmodi:
I - Reflektions- (reflextion light),
II - Durchlicht- (transmitted light), und
III - Auflicht-Verfahren (shine on light).

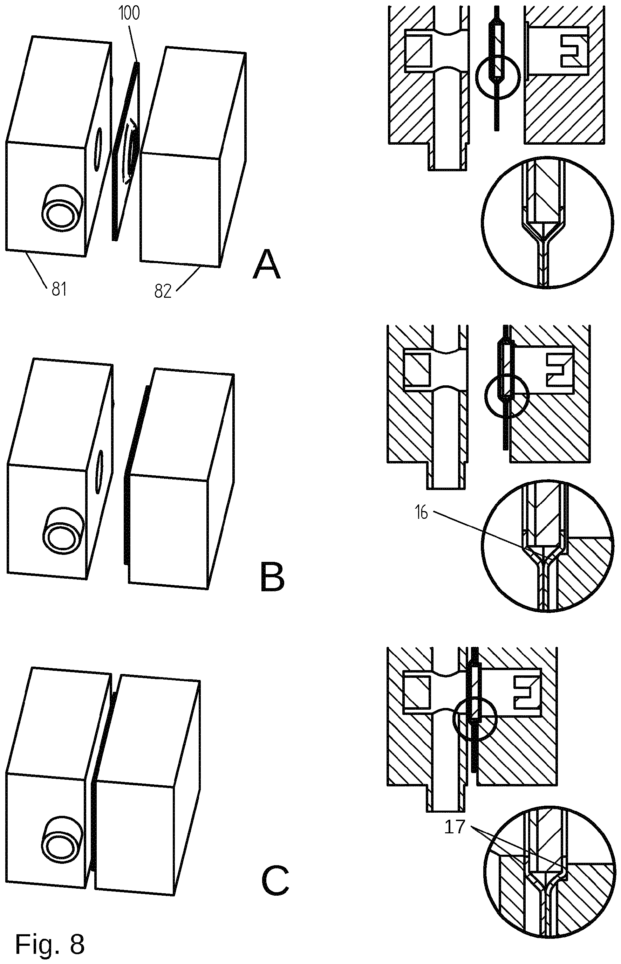

**Fig. 8** stellt die drei Arbeitsschritte A, B und C dar, welche den drei Zuständen "INSERTED", "ADJUSTED" und "FIXED" beim Einspannen des Probenträgers 100 zwischen zwei Modulen 81 und 82 des Messgeräts 80 entsprechen. Wie ersichtlich, ist im ersten Zustand A der Probenträger noch frei beweglich zwischen den beiden Modulen angeordnet. Im zweiten Zustand B liegt der Probenträger 100 an einem der beiden Module an und wird selbsttätig beim einseitigen Kontakt 16 der Auskehlung 15 mit einer korrespondierenden Ausnehmung des entsprechenden Moduls justiert. In dem in der Fig. 8 mit C bezeichneten dritten Zustand schließlich wird unter Einwirken der Andruckkraft 83 (vgl. Fig. 6) eine fluidisch dichtende Verbindung unter Ausbildung eines zweiseitigen fluidisch dichten Kontakts 17 hergestellt zwischen den beiden Modulen und dem Probenträger 100, sodass der Probenträger 100 die von beiden Modulen 81, 82 gebildete Messkammer 90 fluidisch dichtend in zwei voneinander getrennte Bereiche unterteilt.

Erster Zustand, Fig. 8A ("INSERTED"): In den Zwischenraum von zwei gegeneinander verschiebbaren Modulen 81, 82 des Messgerätes 80 ist der Probenträger 100 eingeführt. In diesem Fall sind es links die Baugruppe mit dem Trägergaskanal 84 und auf der rechten Seite die Lichtquelle und der Lichtsensor des zweiten Moduls angeordnet. Es bestehen Spalten zwischen den beiden Messgerätmodulen und dem Probenträger.

Zweiter Zustand, Fig. 8B ("ADJUSTED"): Der Probenträger 100 wird leicht in eine Vertiefung eines der beiden Gerätemodule gedrückt. In diesem Fall befindet sich diese in der Baugruppe mit Lichtquelle 87 und Lichtsensor 88. Die elastischen Schrägen 15 der Folie 10 bewirken eine selbständige Justage des Targets 40.

Dritter Zustand, Fig. 8C ("FIXED"): Der Probenträger 100 ist fest zwischen die beiden Baugruppen eingespannt. Der verbleibende Rand der Folie - im Bild des Verbundes der beiden Folien 10 und 20 - im Bereich der Target-Kante bis zur Öffnung wirkt als Dichtfläche. Die Elastizität der Folienstreifen gleicht Unebenheiten dieser aus und verhindert ungewollten Gasaustausch und den Eintritt von Fremdlicht.

Vorteilhaft gewährleistet der vorgeschlagene Probenträger eine einfache Handhabung, Zeitersparnis beim Wechseln der Targets sowie die Möglichkeit zur lückenlosen, und - beispielsweise in Kombination mit einem angepassten Lesegerät (z.B. einem Barcode-scanner) - auch die automatische Dokumentation von der Herstellung bis zur Verwendung unterschiedlichster Probenträger für unterschiedlichste Proben und unterschiedlichste Anwendungsfelder der vor-Ort-Analyse.

Kurz zusammengefasst umfassen die erzielten Vorteile
1. Einfache Handhabbarkeit der Vorrichtung und des Messverfahrens sowie seiner Verwendung;
2. Möglichkeit automatisierter Fertigung des Probenträgers oder einer Vielzahl von Probenträgern;
3. Möglichkeit einer lückenlosen Dokumentation von Fertigung über Lagerung bis Anwendung und Archivierung;
4. Vereinfachung des Aufbaus eines Messgerätes für VOC durch Wegfall komplizierter Wechselmechanismen;
5. Möglichkeit komplikationsloser Wechsel der Targets unter Feldbedingungen, robuste Arbeitsweise;
6. Durch exakte Positionierung vor einem Mehrfach-Sensor ist die Auswertung unterschiedlicher analytsensitiver Schichten auf einem einzigen Target möglich;
7. Nutzung eines einzigen Probenträgers in verschiedenen Messmodi, beispielsweise im Durchlicht-, Reflektions- und/oder Auflichtverfahren möglich;
8. Möglichkeit eines automatischen Wechsels der Probenträger (z.B. Fluoreszenztargets) und damit ein Dauerbetrieb des betreffenden Sensorsystems sowie einer standardisierbaren Kalibrierung;
9. Nur wenig anfallendes Verschleißmaterial, Wegfall von Dichtungen, daher vorteilhaft umweltfreundlich;
10. Die Herstellung des Probenträgers ist zu geringen Kosten möglich.

Die vorliegende Erfindung wurde anhand von Ausführungsbeispielen erläutert. Diese Ausführungsbeispiele sollten keinesfalls als einschränkend für die vorliegende Erfindung verstanden werden. Die nachfolgenden Ansprüche stellen einen ersten, nicht bindenden Versuch dar, die Erfindung allgemein zu definieren.

### Bezugszeichenliste

- 10: Folie
- 11: Loch
- 15: Auskehlung
- 16: einseitiger mechanischer Kontakt
- 17: zweiseitiger mechanischer Kontakt
- 20: zweite Folie
- 30: starres Substrat
- 40: Target
- 41: analytsensitive Schicht /Sensorschicht
- 50: Transportrand
- 60: Bezeichnung
- 70: Gasfluss, Trägergasstrom
- 80: Messgerät
- 81: Messgerätmodul 1
- 82: Messgerätmodul 2
- 83: Andruckkraft
- 84: Kanal
- 85: Ausnehmung, Kanalausnehmung (AN)
- 86: Messfenster
- 87: Lichtquelle
- 88: Lichtsensor
- 89: Spiegel
- 90: Messraum, Messkammer
- 92: Lichtstrahl
- 100: Probenträger

## Patentansprüche

1. Probenträger (100), umfassend eine erste Folie (10), eine zweite Folie (20) und ein starres Substrat (30), wobei die erste Folie (10) das starre Substrat (30) entlang einer Umfangsrichtung umgibt und der Probenträger (100) eingerichtet ist, eine fluidisch dichtende Verbindung in einem Messraum (90) eines Messgerätes (80) auszubilden und den von zwei Modulen (81, 82) des Messgerätes (80) ausbildbaren Messraum (90) fluidisch dichtend in zwei voneinander getrennte Bereiche zu unterteilen, wobei die zweite Folie (20), benachbart zum starren Substrat (30) auf der ersten Folie (10) befestigt ist und einen Randbereich des starren Substrats (30) überlappt, wobei die beiden Folien an einander gegenüberliegenden Positionen jeweils eine Öffnung (11) aufweisen.

2. Probenträger (100) nach Anspruch 1, wobei die zweite Folie (20) im Randbereich des starren Substrats (30) mit dem starren Substrat (30) verbunden ist.

3. Probenträger (100) nach einem der vorstehenden Ansprüche, wobei ein zentraler Bereich des starren Substrats (30) durch keine der beiden Folien bedeckt ist.

4. Probenträger (100) nach einem der vorstehenden Ansprüche, wobei die erste Folie (10) ein Loch (11) aufweist, das vom starren Substrat (30) verschlossen ist.

5. Probenträger (100) nach einem der vorstehenden Ansprüche, wobei die erste Folie (10) eine Streifenform aufweist und entlang einer Längsrichtung eine Vielzahl von starren Substraten (30) trägt, sodass der Probenträger (100) einen Gurt fortlaufend aufgereihter starrer Substrate (30) bildet.

6. Probenträger (100) nach einem der vorstehenden Ansprüche, wobei die erste Folie (10) einen Transportrand (50) aufweist.

7. Probenträger (100) nach Anspruch 6, wobei der Transportrand (50) eine Perforation, eine sich periodisch wiederholende Folge einer Eindellung, einer Aufwölbung eines Lochs, einer Ausnehmung und/oder einer Verdickung der ersten Folie (10) umfasst.

8. Probenträger (100) nach einem der vorstehenden Ansprüche, wobei ein gemeinsamer Fügebereich beider Folien (10, 20) eine Auskehlung (15) bildet, die das starre Substrat (30) umlaufend säumt, oder die Auskehlung zumindest durch eine der beiden Folien (10, 20) unter Einwirken einer parallel zu einer Flächennormalen des starren Substrats wirkenden Andruckkraft (83) ausbildbar ist.

9. Probenträger (100) nach einem der vorstehenden Ansprüche, wobei
- mindestens eine der beiden Folien chemisch inert ist;
- zumindest eine der Folien (10, 20) ein thermoplastisches Polymer umfasst, ausgewählt unter einem Polyethylen, einem Polypropylen, und/oder einem Polytetrafluoroethylen und/oder einem Polyamid;
- zumindest die das starre Substrat umgebende erste Folie (10) lichtundurchlässig ist, beispielsweise durch Zusatz eines Pigments, bevorzugt durch Zusatz von Ruß.

10. Probenträger (100) nach einem der vorstehenden Ansprüche, wobei eine vom Probenträger getragene Probe eine für einen Analyten sensitive Schicht (41) umfasst.

11. Probenträger (100) nach Anspruch 10, wobei die für den Analyten sensitive Schicht (41) eine Fluoreszenzeigenschaft in Abhängigkeit von einer Art und/oder einer Konzentration des Analyten reproduzierbar ändert und/oder das starre Substrat ein Natriumsilkat- oder Borosilikat-Glas mit der für den Analyten sensitiven Schicht (41) ist.

12. Probenträger (100) nach einem der Ansprüche 1 bis 9, wobei die vom Probenträger (100) getragene Probe ein Depot für eine flüchtige organische Verbindung umfasst.

13. Probenträger (100) nach einem der vorstehenden Ansprüche, wobei der Probenträger eine dem starren Substrat (30) zugeordnete Bezeichnung (60) trägt.

14. Messgerät (80), umfassend zwei beweglich zueinander angeordnete Messgerätmodule (81, 82), die im Kontakt mit einem Probenträger (100) gemäß einem der Ansprüche 1-13 einen Kanal (84) zum Führen eines Fluids und eine Messkammer (90) ausbilden, wobei der Kanal (84) eine Ausnehmung (85) einer Kanalwand aufweist, und die von den zwei Modulen (81, 82) des Messgerätes (80) ausbildbare Messkammer (90) vom Probenträger (100) fluidisch dichtend in zwei voneinander getrennte Bereiche geteilt wird, wobei jeder Teil der fluidisch dichtend geteilten Messkammer (90) zumindest eine optische Komponente umfasst, ausgewählt unter einem Spiegel (89), einer Lichtquelle (87), und/oder einem Lichtsensor (88), wobei die in einem der beiden Messgerätmodule angeordnete Komponente jeweils in Richtung des anderen Messgerätmoduls weist.

15. Messgerät (80) nach Anspruch 14, wobei einer der beiden Teile der fluidisch dichtend geteilten Messkammer (90) ein Messfenster (86) aufweist.

16. Messgerät (80) nach Anspruch 14 oder 15, wobei einer der beiden Messgerätmodule (81, 82) eine Transportvorrichtung für einen gemäß den Ansprüchen 1-13 beschriebenen Probenträger (100) umfasst.

17. Messgerät (80) nach Anspruch 14 bis 16, wobei ein Zusammenwirken der beiden Messgerätmodule (81, 82) mit dem Probenträger (100) einen gasdichten Kontakt zwischen einem im Kanal (84) strömenden Fluid und einer der Ausnehmung (85) zugewandten Seite des Probenträgers (100) ermöglicht.

18. Messgerät (80) nach Anspruch 14 bis 17, wobei die Transportvorrichtung einen diskontinuierlich linearen Vorschub eines starren Substrats (30) in das oder aus dem Messgerät (80) ermöglicht.

19. Messgerät (80) nach Anspruch 18, wobei das Zusammenwirken der beiden Messgerätemodule und des Probenträgers (100) drei Zustände umfasst, und
in einem ersten Zustand der Probenträger (100) frei beweglich zwischen den beiden Messgerätemodulen (81, 82) angeordnet ist,
in einem zweiten Zustand der Probenträger (100) zur Ausnehmung (85) und/oder dem Messfenster (86) justiert angeordnet ist, und
in einem dritten Zustand ein gasdichter Kontakt zwischen der Kanalwand und einer der Ausnehmung (85) zugewandten Seite des Probenträgers (100) herstellbar ist.

20. Analyseverfahren, umfassend:
- Bereitstellen eines in einem Kanal (84) geführten Fluidstromes (70),
- fluidisch dichtendes Kontaktieren eines starren Substrats (30) umfassend eine Probe mit dem Fluidstrom (70), wobei das starre Substrat (30) auf einem Probenträger (100) gemäß den Ansprüchen 1 bis 13 befestigt ist, und eine erste Folie (10) des Probenträgers eine lösbare und fluidisch dichtende Verbindung an einer Kanalausnehmung (85) des Kanals (84) herstellt; und
- qualitatives und/oder quantitatives Bestimmen eines im Fluidstrom (70) vorliegenden Analyten.

21. Analyseverfahren nach Anspruch 20, wobei das Fluid des Fluidstromes (70) ein Gas ist und der Fluidstrom (70) somit ein Trägergasstrom (70) ist.

22. Analyseverfahren nach Anspruch 21, wobei das qualitative und/oder quantitative Bestimmen umfasst:
- Exponieren des starren Substrats (30) mit einer Anregungsstrahlung und Erfassen einer vom starren Substrat (30) abgegebenen Emissionsstrahlung; oder
- Exponieren des den Kanal (84) verlassenden Trägergasstromes (70) gegenüber einem analytsensitiven Sensor, der kalibriert, getestet oder trainiert werden soll.

23. Analyseverfahren nach Anspruch 22, weiter umfassend:
- Temperieren des Trägergasstromes (70) und/oder eines Messgerätmoduls (81, 82) der in Kontakt mit dem Probenträger (100) steht, sodass ein an einer Kanalwandung und/oder am Probenträger (100) adsorbierter Analyt desorbiert und mit dem Trägergasstrom (70) aus dem Kanal (84) getragen wird.

24. Analyseverfahren nach Anspruch 23, wobei eine Temperatur des Trägergasstromes (70) und/oder des starren Substrats (30) in einem Temperaturbereich liegt von
20 bis 75 °C;

25. Analyseverfahren nach Anspruch 22 - 24, wobei die Anregungsstrahlung in einem Wellenlängenbereich liegt von
295 nm bis 800 nm.

26. Analyseverfahren nach Anspruch 25, wobei die Emissionsstrahlung in einem Wellenlängenbereich liegt von
350 nm bis 850 nm.

## Claims

1. A sample carrier (100), comprising a first film (10), a second film (20) and a rigid substrate (30), the first film (10) surrounding the rigid substrate (30) along a circumferential direction, and the sample carrier (100) being configured to form a fluidically sealing connection in a measuring space (90) of a measuring device (80) and to divide the measuring space (90) that can be formed by two modules (81, 82) of the measuring device (80) in a fluidically sealing manner into two regions that are separate from one another, the second film (20) being attached on the first film (10) so as to adjoin the rigid substrate (30) and overlapping an edge region of the rigid substrate (30), the two films each including an opening (11) at mutually opposite positions.

2. The sample carrier (100) according to claim 1, wherein the second film (20) is connected to the rigid substrate (30) in the edge region of the rigid substrate (30).

3. A sample carrier (100) according to any one of the preceding claims, wherein a central region of the rigid substrate (30) is covered by neither of the two films.

4. A sample carrier (100) according to any one of the preceding claims, wherein the first film (10) includes a hole (11), which is closed by the rigid substrate (30).

5. A sample carrier (100) according to any one of the preceding claims, wherein the first film (10) has a strip shape and carries a plurality of rigid substrates (30) along a longitudinal direction, so that the sample carrier (100) forms a belt of continuously stringed rigid substrates (30).

6. A sample carrier (100) according to any one of the preceding claims, wherein the first film (10) comprises a transport edge (50).

7. The sample carrier (100) according to claim 6, wherein the transport edge (50) comprises a perforation, a periodically recurring sequence of an indentation, a bulge of a hole, a recess and/or a thickening of the first film (10).

8. A sample carrier (100) according to any one of the preceding claims, wherein a shared joining region of the two films (10, 20) forms a groove (15), which circumferentially borders the rigid substrate (30), or the groove can be formed by at least one of the two films (10, 20) under the action of a pressing force (83) acting in parallel to a surface normal of the rigid substrate.

9. A sample carrier (100) according to any one of the preceding claims, wherein
- at least one of the two films is chemically inert;
- at least one of the films (10, 20) comprises a thermoplastic polymer, selected from a polyethylene, a polypropylene, and/or a polytetrafluoroethylene and/or a polyamide; and
- at least the first film (10) surrounding the rigid substrate is opaque, for example as a result of the addition of a pigment, and preferably as a result of the addition of carbon black.

10. A sample carrier (100) according to any one of the preceding claims, wherein a sample carried by the sample carrier comprises a layer (41) that is sensitive to an analyte.

11. A sample carrier (100) according to claim 10, wherein the layer (41) that is sensitive to the analyte reproducibly changes a fluorescence property as a function of a type and/or a concentration of the analyte, and/or the rigid substrate is a sodium silicate or borosilicate glass including the layer (41) that is sensitive to the analyte.

12. A sample carrier (100) according to any one of claims 1 to 9, wherein the sample carried by the sample carrier (100) comprises a reservoir for a volatile organic compound.

13. A sample carrier (100) according to any one of the preceding claims, wherein the sample carrier bears a designation (60) that is assigned to the rigid substrate (30).

14. A measuring device (80), comprising two measuring device modules (81, 82) that are movably arranged with respect to one another and, in contact with a sample carrier (100) according to any one of claims 1 to 13, form a channel (84) for guiding a fluid and a measuring chamber (90), the channel (84) including a recess (85) of a channel wall, and the measuring chamber (90) that can be formed by the two modules (81, 82) of the measuring device (80) being divided by the sample carrier (100) in a fluidically sealing manner into two regions that are separate from one another, each part of the fluidically sealingly divided measuring chamber (90) comprising at least one optical component, selected from a mirror (89), a light source (87), and/or a light sensor (88), the component arranged in one of the two measuring device modules in each case pointing in the direction of the other measuring device module.

15. The measuring device (80) according to claim 14, wherein one of the two parts of the fluidically sealingly divided measuring chamber (90) comprises a measuring window (86).

16. The measuring device (80) according to claim 14 or 15, wherein one of the two measuring device modules (81, 82) comprises a transport device for a sample carrier (100) described according to claims 1 to 13.

17. A measuring device (80) according to claims 14 to 16, wherein a cooperation of the two measuring device modules (81, 82) with the sample carrier (100) enables a gas-tight contact between a fluid flowing in the channel (84) and a side of the sample carrier (100) which faces the recess (85).

18. A measuring device (80) according to claims 14 to 17, wherein the transport device enables a discontinuously linear advancement of a rigid substrate (30) into the, or out of the, measuring device (80).

19. The measuring device (80) according to claim 18, wherein the cooperation of the two measuring device modules and the sample carrier (100) encompasses three states, and
in a first state, the sample carrier (100) is freely movably arranged between the two measuring device modules (81, 82);
in a second state, the sample carrier (100) is arranged aligned with the recess (85) and/or the measuring window (86); and
in a third state, a gas-tight contact can be established between the channel wall and a side of the sample carrier (100) which faces the recess (85).

20. An analysis method, comprising:
- providing a fluid flow (70) guided in a channel (84);
- contacting, in a fluidically sealing manner, a rigid substrate (30) comprising a sample with the fluid flow (70), the rigid substrate (30) being attached on a sample carrier (100) according to claims 1 to 13, and a first film (10) of the sample carrier establishing a releasable and fluidically sealing connection at a channel recess (85) of the channel (84); and
- qualitatively and/or quantitatively determining an analyte present in the fluid flow (70).

21. The analysis method according to claim 20, wherein the fluid of the fluid flow (70) is a gas, and the fluid flow (70) is thus a carrier gas flow (70).

22. The analysis method according to claim 21, wherein the qualitative and/or quantitative determination comprises:
- exposing the rigid substrate (30) to excitation radiation, and detecting emission radiation given off by the rigid substrate (30); or
- exposing the carrier gas flow (70) leaving the channel (84) to an analyte-sensitive sensor, which is to be calibrated, tested or trained.

23. The analysis method according to Claim 22, furthermore comprising:
- controlling the temperature of the carrier gas flow (70) and/or of a measuring device module (81, 82) which is in contact with the sample carrier (100), so that an analyte adsorbed at a channel wall and/or at the sample carrier (100) is desorbed and carried out of the channel (84) together with the carrier gas flow (70).

24. The analysis method according to claim 23, wherein a temperature of the carrier gas flow (70) and/or of the rigid substrate (30) is in a temperature range of 20 to 75°C.

25. An analysis method according to claims 22 to 24, wherein the excitation radiation is in a wavelength range of 295 nm to 800 nm.

26. The analysis method according to claim 25, wherein the emission radiation is in a wavelength range of 350 nm to 850 nm.

## Revendications

1. Support d'échantillon (100), comprenant un premier film (10), un deuxième film (20) et un substrat rigide (30), dans lequel le premier film (10) entoure le substrat rigide (30) le long d'une direction circonférentielle et le support d'échantillon (100) est conçu pour réaliser une liaison étanche aux fluides dans un espace de mesure (90) d'un appareil de mesure (80) et pour diviser l'espace de mesure (90), pouvant être constitué de deux modules (81, 82) de l'appareil de mesure (80), de manière étanche aux fluides, en deux zones distinctes, dans lequel le deuxième film (20) est fixé de manière adjacente au substrat rigide (30) sur le premier film (10) et se superpose avec une zone de bord du substrat rigide (30), dans lequel les deux films présentent chacun une ouverture (11) à des positions opposées entre elles.

2. Support d'échantillon (100) selon la revendication 1, dans lequel le deuxième film (20) est relié, dans la zone de bord du substrat rigide (30), avec le substrat rigide (30).

3. Support d'échantillon (100) selon l'une quelconque des revendications précédentes, dans lequel une zone centrale du substrat rigide (30) n'est recouverte par aucun des deux films.

4. Support d'échantillon (100) selon l'une quelconque des revendications précédentes, dans lequel le premier film (10) présente un trou (11) fermé par le substrat rigide (30).

5. Support d'échantillon (100) selon l'une quelconque des revendications précédentes, dans lequel le premier film (10) présente une forme de bande et supporte, le long d'une direction longitudinale, une pluralité de substrats rigides (30), de façon à ce que le support d'échantillon (100) forme une courroie de substrats rigides (30) se succédant de manière continue.

6. Support d'échantillon (100) selon l'une quelconque des revendications précédentes, dans lequel le premier film (10) comprend un bord de transport (50).

7. Support d'échantillon (100) selon la revendication 6, dans lequel le bord de transport (50) comprend une perforation, une séquence périodique constituée d'une indentation, d'un renflement d'un trou, d'un évidement et/ou d'un épaississement du premier film (10).

8. Support d'échantillon (100) selon l'une quelconque des revendications précédentes, dans lequel une zone commune des deux films (10, 20) forme une rainure (15) qui borde le substrat rigide (30) sur sa circonférence, ou la rainure peut être constituée d'au moins un des deux films (10, 20) en appliquant une force de pression (83) agissant parallèlement à une normale à la surface du substrat rigide.

9. Support d'échantillon (100) selon l'une quelconque des revendications précédentes, dans lequel
- au moins un des films est chimiquement inerte ;
- au moins un des films (10, 20) comprend un polymère thermoplastique, sélectionné parmi un polyéthylène, un polypropylène et/ou un polytétrafluoroéthylène et/ou un polyamide ;
- au moins le premier film (10) entourant le substrat rigide est opaque, par exemple grâce à l'ajout d'un pigment, de préférence grâce à l'ajout de noir de carbone.

10. Support d'échantillon (100) selon l'une quelconque des revendications précédentes, dans lequel un échantillon porté par le support d'échantillon comprend une couche sensible à un analyte (41).

11. Support d'échantillon (100) selon la revendication 10, dans lequel la couche sensible à un analyte (41) modifie, de manière reproductible, une propriété de fluorescence en fonction d'un type et/ou d'une concentration de l'analyte et/ou le substrat rigide est un verre de silicate de sodium ou de borosilicate ayant la couche sensible à l'analyte (41).

12. Support d'échantillon (100) selon l'une quelconque des revendications 1 à 9, dans lequel l'échantillon porté par le support d'échantillon (100) comprend un dépôt de composés organiques volatils.

13. Support d'échantillon (100) selon l'une quelconque des revendications précédentes, dans lequel le support d'échantillon comporte une désignation (60) correspondant au substrat rigide (30).

14. Appareil de mesure (80), comprenant deux modules d'appareil de mesure (81, 82) disposés de manière mobile l'un par rapport à l'autre, qui forment, en contact avec le support d'échantillon (100) selon l'une des revendications 1 à 13, un canal (84) pour le transport d'un fluide et une chambre de mesure (90), dans lequel le canal (84) comprend un évidement (85) d'une paroi de canal, et la chambre de mesure (90), pouvant être formée par les deux modules (81, 82) de l'appareil de mesure (80), est divisée par le support d'échantillon (100), de manière étanche aux fluides, en deux zones distinctes, dans lequel chaque partie de la chambre de mesure (90) divisée de manière étanche aux fluides comprend au moins un composant optique, sélectionné parmi un miroir (89), une source de lumière (87) et/ou un capteur de lumière (88), dans lequel le composant disposé dans un des deux modules de l'appareil de mesure est orienté respectivement dans la direction de l'autre module d'appareil de mesure.

15. Appareil de mesure (80) selon la revendication 14, dans lequel une des deux parties de la chambre de mesure (90) divisée de manière étanche aux fluides comprend une fenêtre de mesure (86).

16. Appareil de mesure (80) selon la revendication 14 ou 15, dans lequel un des deux modules de l'appareil de mesure (81, 82) comprend un dispositif de transport pour un support d'échantillon (100) décrit selon les revendications 1 à 13.

17. Appareil de mesure (80) selon les revendications 14 à 16, dans lequel une interaction entre les deux modules de l'appareil de mesure (81, 82) avec le support d'échantillon (100) permet un contact étanche aux gaz entre un fluide s'écoulant dans le canal (84) et un côté du support d'échantillon (100) orienté vers l'évidement (85).

18. Appareil de mesure (80) selon les revendications 14 à 17, dans lequel le dispositif de transport permet une avance linéaire discontinue d'un substrat rigide (30) dans ou hors de l'appareil de mesure (80).

19. Appareil de mesure (80) selon la revendication 18, dans lequel l'interaction des deux modules de l'appareil de mesure et du support d'échantillon (100) comprend trois états et
dans un premier état, le support d'échantillon (100) est disposé de manière mobile librement entre les deux modules de l'appareil de mesure (81, 82),
dans un deuxième état, le support d'échantillon (100) est disposé de manière ajustée par rapport à l'évidement (85) et/ou à la fenêtre de mesure (86), et
dans un troisième état, un contact étanche aux gaz peut être établi entre la paroi du canal et un côté du support d'échantillon (100) orienté vers l'évidement (85).

20. Procédé d'analyse comprenant :
- la mise à disposition d'un flux de fluide (70) guidé dans un canal (84),
- la mise en contact, de manière étanche aux fluides, d'un substrat rigide (30) comprenant un échantillon avec le flux de fluide (70), dans lequel le substrat rigide (30) est fixé sur un support d'échantillon (100) selon les revendications 1 à 13 et un premier film (10) du support d'échantillon établit une liaison amovible et étanche aux fluides avec un évidement de canal (85) du canal (84) ; et
- la détermination qualitative et/ou quantitative d'un analyte présent dans le flux de fluide (70).

21. Procédé d'analyse selon la revendication 20, dans lequel le fluide du flux de fluide (70) est un gaz et le flux de fluide (70) est donc un flux de gaz porteur (70).

22. Procédé d'analyse selon la revendication 21, dans lequel la détermination qualitative et/ou quantitative comprend :
- l'exposition du substrat rigide (30) à un rayonnement d'excitation et mesure d'un rayonnement d'émission émis par le substrat rigide (30) ; ou
- exposition du flux de gaz porteur (70) quittant le canal (84) par rapport à un capteur sensible à l'analyte, qui doit être étalonné, testé ou entraîné.

23. Procédé d'analyse selon la revendication 22, comprenant en outre :
- la régulation de température du flux de gaz porteur (70) et/ou d'un module de l'appareil de mesure (81, 82) qui est en contact avec le support d'échantillon (100), de façon à ce qu'un analyte adsorbé au niveau d'une paroi du canal et/ou du support d'échantillon (100) soit désorbé et transporté hors du canal (84) avec le flux de gaz porteur (70).

24. Procédé d'analyse selon la revendication 23, dans lequel une température du flux de gaz porteur (70) et/ou du substrat rigide (30) se trouve dans une plage de températures de 20 à 75°C.

25. Procédé d'analyse selon la revendication 22 à 24, dans lequel le rayonnement d'excitation se trouve dans une plage de longueurs d'onde de 295 nm à 800 nm.

26. Procédé d'analyse selon la revendication 25, dans lequel le rayonnement d'émission se trouve dans une plage de longueurs d'onde de 350 nm à 850 nm.
